(19) Europäisches Patentamt
European Patent Office
Office européen des brevets

(11) Publication number: **0 112 658 B1**

# (12) EUROPEAN PATENT SPECIFICATION

(45) Date of publication of patent specification: **07.08.91**

(51) Int. Cl.[5]: **A61K 31/02**, A61K 31/025, A61M 35/00

(21) Application number: **83307220.0**

(22) Date of filing: **25.11.83**

## (54) Extraocular device for treating the eye.

(30) Priority: **26.11.82 US 444850**

(43) Date of publication of application:
**04.07.84 Bulletin 84/27**

(45) Publication of the grant of the patent:
**07.08.91 Bulletin 91/32**

(84) Designated Contracting States:
**BE CH LI NL SE**

(56) References cited:
**EP-A- 0 089 232**
**EP-A- 0 089 815**
**WO-A-81/00002**

(73) Proprietor: **CHILDREN'S HOSPITAL MEDICAL CENTER**
**Elland and Bethesda Avenues**
**Cincinnati Ohio 45229(US)**

(72) Inventor: **Clark, Leland C., Jr.**
**218 Greendale Avenue**
**Cincinnati, OH 45220(US)**

(74) Representative: **Allam, Peter Clerk et al**
**LLOYD WISE, TREGEAR & CO. Norman House 105-109 Strand**
**London WC2R 0AE(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 112 658 B1

## Description

Background of the Invention

Opthalmic preparations are administered into the eye for treating a wide variety of ocular disorders. Usually, these preparations are sterile products designed for either topical application onto the internal eyelid or instillation into the cul-de-sacs strategically positioned between the eyeball (cornea and bulba conjunctiva) and eyelids (palpebral conjunctiva). Opthalmic preparations may also be prepared in the form of an injection. At the present time, the following are the most common methods of delivering therapeutic substance to the eye: by the mechanism of some sort of vehicle or delivery system to the surface of the eye comprising ocular solutions, suspensions, ointments, gels and inserts; and by either periocular or systemmic injections. If there are any diseases within the eye to be treated pharmaceutically, however, the obviously safe and most preferred method of providing that treatment would be by extraocular topical application. Such disorders include dry eye syndrome caused by keratoconjunctivitis sica, tear abnormalities, atrophy of the lacrimal gland, ocular pemphigoid, chemical burns, chronic keratoconjunctivitis, corneal epithelial diseases (corneal ulcers, recurrent corneal erosion and marginal ulcers), and corneal vascularization due to corneal injury, infection or transplantation.

Notwithstanding the safety and apparent convenience of topical ophthalmic preparations, several have significant disadvantages which adversely affect their suitability and efficacy. Some of the disadvantages are brief duration in the cul-de-sacs, irritation, burning or stinging sensations, stickiness, and discomfort, all of which can be attributed to the vehicle, preservatives, inserts or drug itself. Because of these disadvantages, patients may not even comply with their doctor's advice for using them.

Presently, all ophthalmic preparations, including those commonly referred to as "eye drops", are required to be sterile before their instillation into the eye. In most ophthalmic preparations, sterility is achieved and maintained by incorporating preservatives. Preservatives, however, are known to adversely affect the surface structure of the corneal epithelium and disrupt the microvilli which are necessary for the cornea to retain the tear film layer. Thus, the mucin layer of the tear film cannot be properly adsorbed onto the abnormal microvilli of the corneal and conjunctiva surfaces. Without adsorption of the mucin layer onto the corneal and conjunctiva surfaces, the wetting of these surfaces by aqueous tear film layer is prevented.

In addition to the sterility requirement, eye drops, comprising suspensions and solutions, are rapidly eliminated from the cul-de-sacs by the naso-lacrimal drainage system. As a result of their aqueous properties and low specific gravity, drops are miscible with the secretory liquids of the eye subjecting them to a faster rate of explusion by way of the blinking mechanism. Unfortunately, their persistence in the cul-de-sacs is limited thereby significantly compromising suitability and efficacy. Thus, to achieve therapeutic results, ophthalmic eye drops require frequent administration. Because of the physical properties of suspensions, they tend to remain longer in the cul-de-sacs than solutions. Nevertheless, particles are dispersed throughout suspensions which are severely irritating to the corneal and conjunctiva surfaces.

Ophthalmic ointments and gels also cause undesirable effects when applied to the internal eyelid. Upon application, they produce a film over the corneal and conjunctiva surfaces blurring vision while usually failing to deliver a uniform dose. Moreover, they may interfere with the preocular tear film precluding its attachment to the corneal and conjunctiva surfaces. Periocular and systemmic injections require an ophthalmologist not only to administer but to monitor the drug. Injections tend to cause the patient a great deal of pain and discomfort, anxiety, and inconvenience. Eye inserts provide a delivery system that maintains a low but uniform and constant delivery of a drug. Since the drug is delivered for the duration, frequent administration, as with drops, is not necessary. The major disadvantages associated with ocular inserts involve unnoticed loss of the insert from the eye, slippage of the insert into the area of sight, rupturing of the insert's membrane subjecting the delicate structures of the eye to excessive medication, possible tachyphylaxis, and structural changes in the ciliary muscle resulting from the constant exposure to the drug. Furthermore, there is some degree of manual dexterity required of the patient to position the insert therapeutically and strategically correct.

With respect to corneal epithelial disease, eg corneal ulcers and marginal corneal ulcers, there is a breakdown of the epithelial barrier which may result in infectious diseases and chronic recurrent breakdowns. The cornea, due to these disorders, will increase in hydration and loss of transparency. Thus, there is a need for proper ophthalmic preparations that can promote rapid and proper healing of such diseases to avoid formation of corneal scars or opacities. Corneal transplantation or repair is indicated when the cornea has become opaque, infiltrated, or diseased. Because the healing process, as stated above, results in corneal opacification and scars, normal vision can be partially or totally obstructed. In general, corneal transplantation or repair increases hydration while decreasing oxygenation of the cornea. Thus, an ingrowth

of vessels is usually seen within the cornea after repair or transplantation responding to the corneal scar tissue, increase in hydration and lack of oxygen. Unfortunately, corneal repair or transplantation, as with corneal epithelial disorders, can occlude normal vision.

It has heretofore been known to immerse experimental animals in liquid perfluorocarbons saturated with oxygen and the experimental animal is actually capable of breathing the liquid. For instance, in 1966, Leland C. Clark, Jr. and his co-worker F. Gollan, published such matters in Science 152, 1755-1756 (1966). In addition, photographs of a mouse breathing a liquid perfluorocarbon saturated with oxygen are provided in Angewandte Chemie, 1978, pp 621-700 at p. 622. During such experiments, the eye of the experimental animal was exposed to a perfluorocarbon liquid, however, there was no appreciation or intention of treating a disorder of the eye of the experimental animal with such perfluorocarbon liquid. Accordingly, to applicant's knowledge herein, heretofore there has been no knowledge or disclosure of treating disorders of the eye by the external administration of perfluorocarbons.

It is apparent from the above brief overview of various ophthalmic preparations or devices for the eye in pathological processes and the current state of knowledge that there are vital needs for more suitable, efficacious and advantageous extraocular ophthalmic preparations.

We have now discovered that liquid perfluorocarbons and substituted derivatives thereof are useful as a therapeutic agent or vehicle in extraocular ophthalmic preparations. These liquids act as lubricating, wetting or protective agents, as well as vehicles for other substances or drugs. These liquids will also remain within the cul-de-sacs for greater periods of time since they have high specific gravities. In their pure state, these liquids are not aqueous, wherefore isotonicity and pH do not need to be considered. Most fluorocarbon neat liquids have a refractive index near that of the aqueous, vitreous and tears. Moreover, preservatives are not required to be incorporated into such liquids to maintain sterility. Corneal and conjunctiva surfaces, when treated with such liquids, are provided with greater amounts of oxygen because of high oxygen properties of the perfluorocarbons.

Thus, the present invention provides a device for the extraocular treatment of the eye, comprising a container, a perfluorocarbon or substituted derivative thereof in liquid form within the container, and means for dispensing controlled amounts of said perfluorocarbon or substituted derivative thereof extraocularly onto the eye to be treated.

As indicated above, perfluorocarbons have been found to be advantageous agents when instilled extraocularly into the eye as well as a possible vehicle for other drugs and agents. The hydrophobic and lipophobic properties of these liquids have been found particularly useful as extraocular agents. Perfluorocarbon liquids have been instilled extraocularly into the eye of an experimental animal as well as a human to function as lubricants or wetting solutions. They have been proven to be useful ophthalmic solutions, and the eyes of experimental animals or humans treated extraocularly with this liquid maintained normal vision and demonstrated no adverse effects. Furthermore, the high specific gravity of the perfluorocarbon liquids permits their retention within the cul-de-sacs for a greater period of time. These and other remarkable discoveries will become further understood in the details which follow herein below.

The perfluorocarbon liquids preferably employed are transparent or light transmissive, inert, and remain viscous indefinitely. This invention is predicated in part upon the discovery that such perfluorocarbon liquids are ideal in extraocular preparations. Moreover, they dissolve oxygen and carbon dioxide extremely well, and can be sterilized by autoclaving. Thus, these liquids are comprised of unusual chemical and physical properties endowing them with unique, unexpected and advantageous uses in extraocular ophthalmic preparations. Another important discovery involved in this invention is that these perfluorocarbons can be instilled into the cul-de-sacs and will remain therein for greater periods of time because of their greater density and immiscibility with aqueous media. Also, it is found that upon blinking, a microemulsion of the perfluorocarbon liquid may develop within the lipid layer of the preocular tear film providing significant lubricating and wetting properties for the corneal and conjunctiva surfaces. Remarkably, these "microemulsions" remained in contact with the external surfaces of the eye for a substantial period of time. Finally, the external surface of the eye will be better oxygenated because of the perfluorocarbon's high diffusion properties for oxygen and carbon dioxide.

For instance, the liquid perfluorocarbon can be applied to enhance oxygenation of the cornea and lubricate the epithelial surface to reduce adverse side effects associated with the contact lens use. In addition, the liquids can be applied as a protective agent in corneal epithelial diseases to prevent infection and chronic recurrent epithelial breakdowns. In corneal repair, transplantation or disease, the liquids enhance oxygenation of the corneal tissue and decrease hydration thereby reducing superficial vascularization, scar tissue and opacities. As a vehicle, the liquids can deliver pharmaceutical agents to treat extraocular or intraocular disorders.

The perfluorocarbons and derivatives thereof suitable for use in this invention are in liquid form. The

term "liquid", as used herein, is a comprehensive designation incorporating compounds that are in a state neither solid or gaseous such as liquids, suspensions, emulsions and gels. The term "perfluorocarbon" means a "cyclic" or "acyclic" compound of carbon, whereas the term "substituted derivatives thereof" characterizes substituted perfluorocarbons with chemical elements within their structures such as oxygen, nitrogen and bromine, etc. It should also be noted that the term "perfluorocarbon" denotes substitution of all hydrogen atoms, or substantially all, attached to the carbon atom chain or ring and any carbon side groups with fluorine. It is conceivable in the manufacture of such compounds that minor amounts of substantially fluorinated derivatives may be mixed with completely fluorinated compounds. This is permissible providing that the lack of complete replacement of all hydrogens does not affect the essential characteristics of the liquid perfluorocarbons of this invention, particularly when active hydrogens critically enhance the toxicity of the compounds. Perfluoropolymers such as E3 or E4, identified hereinafter, do have one hydrogen, but are still satisfactory. Among the perfluorocarbon compounds which may be employed are perfluorotributylamine (FC47), perfluorodecalin (PP5), perfluorotetrahydrofuran (FC80), perfluoroether (PID) $[(CF_3)_2CFOCF_2(CF_2)_2CF_2OCF(CF_3)_2]$,

perfluoroether (PIID) $[(CF_3)_2CFOCF_2(CF_2)_6CF_2OCF(CF_3)_2]$,

perfluoropolymer (E3) $[CF_3CHF(OCF_2\overset{CF_3}{C}F)_2OCF_2CF_2CF_3]$,

perfluoropolymer (E4) $[CF_3CHF(OCF_2\overset{CF_3}{C}F)_3OCF_2CF_2CF_3]$,

perfluoroetherpolymer (Fomblin Y/01), perfluorododecane, perfluorobicyclo[4.3.0.]nonane, perfluorotrimethylcyclohexane, perfluoroisopropylcyclohexane, perfluoroendotetrahydrodicyclopentadiene, perfluoroadamantane, perfluoroexo-tetrahydrodicyclopentadiene, perfluorobicyclo[5.3.0.]decane, perfluorotetramethylcyclohexane, perfluorool-1-methyl-4-isopropylcyclohexane, perfluoro-n-butylcyclohexane, perfluorodimethylbicyclo[3.3.1.]nonane, perfluoro-1-methyl adamantane, perfluoro-1-methyl-4-t-butylcyclohexane,perfluoro-decahydroacenaphthene, perfluorotrimethylbicyclo[3.3.1.]nonane, perfluoro-n-undecane, perfluorotetradecahydrophenanthrene, perfluoro-1,3,5,7-tetramethyladamantane, perfluorododecahydro fluorene, perfluoro-1,3-dimethyl adamantane, perfluoro-n-octylcyclohexane, perfluoro-7-methyl bicyclo[4.3.0.]nonane, perfluoro-p-diisopropylcyclohexane, and perfluoro-m-diisopropylcyclohexane, perfluorooctyl bromide, and perfluoro 1-bromobutylisopropyl ether.

It is to be understood that perfluorocarbon liquids of this invention may be formed of "neat" perfluorocarbon liquids, or of emulsions, suspensions or solutions of perfluorocarbons in mixture with themselves or other solvents. For instance, perfluoro-1,3-dimethyl adamantane is normally a solid but in mixture with perfluorotrimethylbicyclo[3.3.1.]nonane a liquid is formed, i.e. DAWN. The neat liquids are preferred because of their inertness and non-aqueousness. Also, when the perfluorocarbon liquids are emulsified in water, sometimes milky or even somewhat clear or transparent liquids, emulsions, gels or solutions can result which are suitable for use in this invention.

In extraocular ophthalmic preparations fluid transparency is preferred, but even somewhat milky fluids may be used.

In brief, then, the nature of the "liquid" state may include pure liquid perfluorocarbon, emulsions, solutions, suspensions, etc, of perfluorocarbon compounds in other liquid mediums. Incorporated herein by reference, therefore, are emulsions or suspensions of perfluorocarbons disclosed in U.S. Patents 3,911,138 and 4,105,798 as suitable liquids for use in this invention. Perfluorocarbons are capable of being synthesized by well known chemical or electrochemical processes.

The chemical processes yield fairly pure substances of known structure, having well defined boiling points. Whereas the electrochemical processes tend to yield a mixture of isomers, the liquids have well defined boiling points. With respect to gas chromatography, the liquid is capable of being well defined by either the packed or capillary column procedure. The standard to define each compound in gas chromatography is prepared as follows: 2 microliters of neat liquid are added to 120 milliliters of air in a sealed bottle and allowed to vaporize producing a stock standard; upon vaporization, 120 microliters of the vapor from the stock standard are added to another 120 milliliters of air in a sealed bottle producing the working standard; the sample measured by the procedure is withdrawn from the working standard, thus, a typical sample will contain 16.7 pico liters of perfluorocarbon per milliliter of standard; however, in the capillary column procedure, the sample is split into a ratio of 23:1, therefore, only 1/23 of the sample is actually measured.

As indicated in Table II, the retention time is highly definitive of the liquid used in this invention. Moreover, the capillary procedure is more specific than the packed column procedure by defining additional characteristic peaks of the compound. Thus, a more precise definition of the compound can be had with the capillary column procedure as exemplified for perfluoro 1-methyldecalin in the following TABLE.

TABLE I

Gas Chromatography*

| | Packed Column** | Capillary Column**** |
|---|---|---|
| **Set Up** | | |
| Standard | [16.7 pl/ml]**** | [16.7 pl/ml]**** |
| Recorder Sensitivity | 0.001γ full scale | 0.001γ full scale |
| Column Temperature | 100°C | 37°C |
| Detector Temperature | 250°C | 250°C |
| Injector Temperature | 150°C | 150°C |
| $N_2$ Gas Flow | 40 ml/min | 40 ml/min |
| Split | --- | 23:1 |
| Recorder Speed | 2.5 cm/min | 2.5 cm/min |
| **Compound** | | |
| PP9 (perfluoro 1-methyldecalin) | | |
| Attenuation | 8 | 4 |
| Sample | 50 mcl | 100 mcl |
| Peaks | 3 | 7 |
| Retention Time | | |
| $Peak_1$ | 124.8 sec | 211.2 sec |
| $Peak_2$ | 136.8 sec | 240 sec |
| $Peak_3$ | 196.8 sec | 340.8 sec |
| $Peak_4$ | --- | 362.4 sec |
| $Peak_5$ | --- | 379.2 sec |
| $Peak_6$ | --- | 391.2 sec |
| $Peak_7$ | --- | 403.2 sec |

* Antek 300 Gas Chromatograph instrument
** Supelco, Inc. Packed Column
*** Scientific Glass Engineering Capillary Column
**** pl/ml = picoliters/milliliter

Preferred liquid perfluorocarbons, exemplified by perfluoro-1-methyl decalin, all have in common a high solubility in oxygen and carbon dioxide, inertness, high density, and transparency. They are suitable for instillation into the eye as extraocular ophthalmic preparations and in the treatment of ophthalmologic disorders. A particular perfluorocarbon or a mixture of perfluorocarbons falling within the family of liquids

exemplified by the above derivative may be used according to the principles of our invention. One main property generic to the preference of the liquids according to this invention over other fluoro-containing compounds is their chemical structure rendering them RES-phobic. These compounds have been defined in U.S. Patent 3,911,138 as "perfluorocyclocarbons", especially perfluoro (methylcyclohexane), perfluoro-1-methyldecalin [also known as perfluoro(decahydro-1-methylnaphthalene)], perfluoro (1,3-dimethylcyclohexane), perfluoro (decahydronaphthalene), and perfluoro (decahydrodimethylnaphthalene), or mixtures thereof, perfluorinated bicyclononane, perfluorinated bicyclooctane, perfluorinated adamantane hydrocarbon, perfluoromethyladamantane and perfluorodimethylbicyclo[3.3.1.]nonane, perfluorodimethyladamantane and perfluorotrimethylbicyclo[3.3.1.]nonane, perfluorotetrahydrodicyclopentadiene and perfluorobicyclo[5.3.0]-decane, perfluorotetrahydrodicyclopentadiene, perfluorinated bicyclononane, perfluorinated bicyclooctane, perfluorinated adamantane hydrocarbon, perfluoromethyladamantane and perfluorodimethylbicyclo[3.3.1]-nonane, perfluorodimethyladamantane and perfluorotrimethylbicyclo[3.3.1.]nonane, and perfluorotetrahydrodicyclopentadiene and perfluorobicyclo[5.3.0]decane. RES-phobic perfluorinated liquids tend to accumulate less in the bodies of animals, principally in the liver, and to a lesser extent in the speen and kidneys. This is significant because such compounds will not become fixed indefinitely within the cells of the organ. There is another property associated with this class of perfluorocarbons that is preferentially utilized when they are introduced into the eye. A perfluorocarbon or a mixture thereof is preferably employed having a vapor pressure within the range of about 1 to about 3,3 kPa (25 torrs) at about 35°C. Thus, such liquids or mixtures are not only RES-phobic, but upon escaping the cell expediently, they will not cause adverse gas collection in the tissue of animals.

In its broadest application, the invention involves the extraocular employment of a perfluorocarbon in a liquid state as an extraocular ophthalmic preparation to treat ocular disorders. The liquid is placed or instilled in the eye extraocularly by a device comprising a container for the liquid and means for dispensing controlled amounts of the liquid from the container. Such a device can, for example, take the form of an eye dropper such as a dropper bottle, or a separate dropper or pipette may be employed. In this connection, due to the specific gravity, low surface tension and high vapor pressure of the specific perfluorocarbon liquids, it may be necessary that the means for dispensing the liquid extraocularly onto the eye in a controlled manner should provide a fine dispensing aperture, or a sponge-like dispensing conduit, or include other means which permits controlled dispensing of the liquid, which otherwise may run too freely from its container. Other means necessary to instil a gel or ointment may be used. For example, the neat liquid or gel can be dropped into the inferior cul-de-sac, the space between the eyeball and eyelid, by tilting the head back and gently pulling down the inferior palpebral conjunctiva until a "V" pocket is formed between the eye and the inferior eyelid, whereupon the liquid or gel can be instilled into the "V" pocket. The objective is to instill the neat liquid or gel into the inferior formix of the inferior cul-de-sac. Because of the density, viscosity, and immiscibility of the perfluorocarbons, such liquids will sink to and remain at the inferior formix. The mechanism of blinking will then act to disperse the liquids across the corneal and conjunctiva surfaces. The liquids, as a direct result of their immiscibility, will usually disperse as "microemulsions" rather than solutions. Microemulsions will be dispersed within the lipid layer of the preocular tear film acting to lubricate and wet the epithelial surfaces with a substantial amount remaining in the cul-de-sacs for significant periods of time being available for continuous dispersion. The significant retention of the liquids within the cul-de-sacs occurs as a result of their immiscibility, density and viscosity.

The eye utilizes the mechanism of blinking to not only spread the fluids therein, but also to eliminate such fluids by way of the lacrimal canaliculi. That is, the drainage of fluid, such as tears, does not depend upon gravity for its elimination, but rather, the fluid enters the puncta and passes along the lacrimal canaliculi by capillary attraction aided by aspiration caused by a contraction of muscle embedded within the eyelids. When the eyelids are partially closed, as in the initial phase of blinking, contraction of the muscles causes the puncta to close. The canaliculi are then compressed while the lacrimal sac is dilated when the eyelids are further closed. Fluid, thus, is aspirated into the sac and forced down the nasolacrimal duct toward its aperture into the nose. When a perfluorocarbon liquid is dropped into the eye, the mechanism of blinking is less efficient in its elimination. This effect is due to the density, viscosity, and immiscibility of these liquids which act to antagonize the aspiration effect of blinking, retarding their expulsion. Perfluorocarbon liquids, as extraocular ophthalmology preparations, are beneficial for treating infections, cornea transplants or repairs, and dry eyes and tear abnormalities such as aqueous tear deficiency, tear film instability, surfacing problems, effects or agents, and tear film drainage. Upon dispersion of the perfluorocarbon liquids across the corneal and conjunctiva surfaces, the microemulsions are dispersed within the lipid or outermost layer of the tear film where it will primarily demonstrate its effect. There should be little if no adverse effects on the mucin layer, as seen with ointments, when such liquids are instilled. Thus, the instillation of perfluorocarbon liquids as extraocular ophthalmology preparations will provide a lubricating, wetting or protecting surface without interferring with mucin adsorption onto the corneal and conjunctiva surfaces. Thus, the liquids will not only provide these beneficial effects on the pre-ocular tear film, but will remain within the cul-de-sacs for longer periods of time as a result of their immiscibility, density and viscosity. In contrast to these advantageous liquid perfluorocarbons, the present ocular lubricants, comprising solutions of hydroxypropyl methylcellulose, polyvinyl alcohol, polyvinyl pyrrolidine soluble polymers, dextran and mixtures thereof, are rapidly cleared from the eye requiring frequent administration.

No preservatives are necessary to maintain sterility in the preferred preparations of the invention because neat liquid perfluorocarbons are nonaqueous. Thus, the microvilli within the epithelial of the cornea are not adversely effected, as exhibited by present extraocular ophthalmology preparations, and the adsorption of mucin onto the microvilli remain intact. Since the liquids are non-aqueous, the pH or isotonicity does not have to be considered to avoid irritation and stinging upon their instillation. With contact lenses, the liquids can be employed as protective, wetting, lubricating and oxygenating extraocular ophthalmic preparations. The irritation and possible superficial vascularization and opacification of the cornea will also be reduced. In corneal epithelial diseases, such as corneal ulcers, recurrent corneal erosion and marginal corneal ulcers, the liquid perfluorocarbon preparations will decrease the incidence of corneal vascularization, scarring and opacification as seen with such corneal disorders. Likewise, in corneal repair and/or transplantation, the application of perfluorocarbon liquids during and after such procedures should decrease or eliminate the untoward side effects of corneal vascularization, scarring and opacification. It is the oxygen-carrying property of the liquid perfluorocarbons as well as their non-aqueous, viscous, and dense properties that make them ideal liquids to treat such procedures.

Due to the retention of perfluorocarbon liquids within the cul-de-sacs for considerable periods of time, such liquids can be employed as suitable vehicles for other substances, such as pharmaceuticals to treat extraocular and intraocular disorders. Presently drops, ointments, and ocular inserts are utilized to deliver drugs extraocularly. As stated above, solutions are readily excreted, suspensions are irritating, ointments blur vision and interfere with mucin adsorption, while ocular inserts are uncomfortable and subject to rupture or loss. With the use of liquid perfluorocarbons as vehicles, the number of administrations can be reduced while the adverse effects of ointments, suspensions and inserts can be eliminated.

In view of the above description, it is apparent that perfluorocarbon liquids and derivatives thereof are unique and advantageous liquids when used in extraocular ophthalmological preparations. The invention, its principles and objectives will be further understood in view of the following examples with reference to the drawings which are anatomical illustrations of the eye. Fig. 1 constitutes a vertical section of the eye illustrating the eyelid, and the external and internal components of the eye. As stated, the secretory components of the eye secrete the preocular tear film which is comprised of three layers. The oil layer originates from the meibomian glands and glands of Zeis, the aqueous layer is produced by the lacrimal and accessory lacrimal (Wolfring's and Krause) glands, while mucin is secreted by the goblet cells (not illustrated) which are scattered over the palpebral conjunctiva. The superior and inferior formix constitute the deposit area within the superior and inferior cul-de-sacs. The middle layer, the tear fluid, is aqueous and provides hydration, antibacterial agents and nutritional elements to the external surfaces. The innermost layer of the tear film comprises mucin which covers the conjunctiva and corneal surfaces. Mucin functions to permit the overlying aqueous layer to spread across the surfaces. Herein the term "external surface" of the eye is meant to include broadly all such surfaces shown in the drawing and referred to herein which may come into direct contact with liquid which is introduced between an eyelid and eyeball.

Fig. 2 illustrates the excretory apparatus employed by the eye to eliminate fluids therein. The puncta are the apertures leading into the lacrimal canaliculus and eventually into the nasal lacrimal duct.

EXAMPLE I

A single drop from a 3 ml syringe and a 25 gauge needle containing perfluoro 1-methyldecalin (PP9) was instilled, ie placed into each eye of a rabbit. The rabbit was observed for a period of two hours through a Codman-Mentor Operating Microscope. Specifically, the examination of the rabbit cornea showed no evidence of corneal toxicity as manifested by the lack of epithelial staining and maintenance of corneal transparency. Also, there was no inflammation or irritation observed in the bulbar or palpebral conjunctiva. Further, the rabbit demonstrated no abnormal visual responses.

EXAMPLE II

A single drop containing perfluoro-1-methyldecalin (PP9), was instilled into the right eye of a human. The instillation, using a plastic dropper bottle, was made onto the cornea of the right eye. Prior to and after

instilling the liquid, various tests including Visual Acuity, Corneal Sensitivity, Anterior Segment Examination with and without Fluoroscein, and Tear Film stability were conducted for comparative purposes. No negative effects or results were found before or after the instillation of the perfluoro-1-methyldecalin. Thus, the results demonstrated that the eye remained unchanged after the experiment. The subject's visual acuity, as measured before and after, was 20/20 corrected. There was no change in his corneal sensitivity. The slit-lamp biomicroscopy test with and without fluorescein detected no corneal ulcers, irritations or drying cells on his ocular surface. The subject's tear stability or break-up time, also measured before and after, remained the same at 25 seconds. Furthermore, the subject experienced no gritty, stinging, or burning sensations, or excess mucous secretions from the instilled liquid. The subject found the liquid to be comfortable, soothing and did not interfere with his blinking or vision. The liquid was detected in his eye for up to an hour after it had been instilled. Finally, there were no ocular, nasal, or gastrointestinal side effects experienced by the subject.

EXAMPLE III

A rabbit, having a Pasturella infection in his right eye was treated with an extraocular ophthalmologic perfluorocarbon preparation according to this invention. The rabbit was treated on Days 0, 1, 3, 6 and until Day 7 by dropping a therapeutic ophthalmic emulsion containing PP9 (perfluoromethyl decalin identified above) as an antibiotic and antifungal preparation. The composition of the therapeutic ophthalmic solution was 10 cc PP9, 10 cc PANALOG, 10 cc sterile water and 30 cc pluronic F68 (8 g/dl). The mixture was sonicated to make a white gel emulsion. PANALOG (Squibb) is a nystatin, neomycin sulfate, thiostrepton, triamcinolone acetonide ointment. Each ml contains nystatin (100,000 units), neomycin sulfate equivalent to neomycin base (2.5 mg), thiostrepton (2,500 units), triamcinolone acetonide (1 mg in PLASTIBASE (plasticized hydrocarbon gel), a polyethylene and mineral oil gel base.

After 7 days of treatment, the infection caused by the Pasturella was completely gone. Photographs and cultures were taken on day 7 and it was observed that there was some redness of the conjunctiva which was minor. The emulsion preparation herein could be improved by pH and ionic adjustment if desired.

Registered trademarks are accordingly acknowledged

## Claims

1. A device for the extraocular treatment of the eye, comprising a container, a perfluorocarbon or substituted derivative thereof in liquid form within the container, and means for dispensing controlled amounts of said perfluorocarbon or substituted derivative thereof extraocularly onto the eye to be treated.

2. A device according to Claim 1, wherein said liquid is a perfluorocyclocarbon.

3. A device according to Claim 2, wherein said perfluorocyclocarbon is selected from perfluoro-(methylcyclohexane), perfluoro(1,3-dimethylcyclohexane), perfluoro(decahydronaphthalene), perfluoro-(decahydro-1-methylnaphthalene),perfluoro(decahydrodimethylnaphthalene), perfluorodimethyladamantane, perfluorotrimethylbicyclo[3.3.1]nonane, perfluorotetrahydrodicyclopentadiene, perfluorobicyclo-[5.3.0]decane and perfluorodimethylbicyclo[3.3.1.]nonane, perfluorooctyl bromide, perfluoro 1-bromobutylisopropyl ether, and mixtures thereof.

4. A device according to any preceding claim, wherein said perfluorocarbon is in the form of a neat liquid.

5. A device according to any one of Claims 1-3, wherein said perfluorocarbon is in the form of an emulsion or gel.

6. A device according to any preceding claim, wherein said liquid also contains a pharmaceutical agent.

7. A device according to any preceding claim, constructed in the form of an eye dropper.

## Revendications

1. Dispositif de traitement extraoculaire de l'oeil, comportant un réservoir, du perfluorocarbone ou un substitut dérivé en forme liquide dans ledit réservoir, et un distributeur permettant le dosage contrôlé

dudit perfluorocarbone ou substitut dérivé de façon extraoculaire sur l'oeil à traiter.

2. Dispositif selon la revendication 1, dont le liquide est un perfluorocyclocarbone.

3. Dispositif selon la revendication 1, dont ledit perfluorocyclocarbone est sélectionné à partir de perfluor (méthyle cyclohexane), perfluoro (1,3-diméthylecyclohexane), perfluoro (decahydronaphtalène), perfluoro(decahydro-1-méthyle naphtalène), perfluoro diméthyle adamantane, perfluorotriméthylebicyclo[3.3.1]nonane, perfluorotetrahydrodicyclopentadiène, perfluorobicyclo[5.3.0]-decane et perfluorodiméthylebicyclo[3.3.1]nonane, perfluoro octyle bromure, perfluoro 1-bromobutylisopropyle ether et leurs mélanges.

4. Dispositif selon toute revendication antérieure, dont ledit perfluorocarbone est sous forme de liquide pur.

5. Dispositif selon l'une ou l'autre des revendications 1 à 3, dont ledit perfluorocarbone est sous forme d'émulsion ou de gel.

6. Dispositif selon toute revendication antérieure, dont ledit liquide contient également un produit pharmaceutique.

7. Dispositif selon l'une des revendications ci-avant, construit sous forme de compte-gouttes.

**Patentansprüche**

1. Einrichtung zur extraokulären Behandlung des Auges, umfassend einen Behälter, einen Perfluorkohlenstoff oder ein substituiertes Derivat davon in flüssiger Form in dem Behälter, und eine Vorrichtung zur Abgabe von kontrollierten Mengen des Perfluorkohlenstoffs oder des substituierten Derivats davon auf das zu behandelnde Auge.

2. Einrichtung nach Anspruch 1, worin die Flüssigkeit ein Perfluorcyclokohlenstoff ist.

3. Einrichtung nach Anspruch 2, worin der Perfluorcyclokohlenstoff von Perfluor(methylcyclohexan), Perfluor(1,3-dimethylcyclohexan), Perfluor(decahydronaphthalin), Perfluor(decahydro-1-methylnaphthalin, Perfluor(decahydrodimethylnaphthalin), Perfluordimethyladamantan, Perfluortrimethylbicyclo[3.3.1]-nonan, Perfluortetrahydrodicyclopentadien, Perfluorbicyclo[5.3.0]decan und Perfluordimethylbicyclo-[3.3.1]nonan, Perfluoroctylbromid, Perfluor 1-Brombutylisopropyläther und Mischungen davon gewählt wird.

4. Einrichtung nach einem der vorhergehenden Ansprüche, worin der Perfluorkohlenstoff in der Form einer klaren Flüssigkeit ist.

5. Einrichtung nach einem der Ansprüche 1 bis 3, worin der Perfluorkohlenstoff in der Form einer Emulsion oder eines Gels ist.

6. Einrichtung nach einem der vorhergehenden Ansprüche, worin die Flüssigkeit auch einen pharmazeutischen Wirkstoff enthält.

7. Einrichtung nach einem der vorhergehenden Ansprüche, die in der Form eines Augentropfers gebildet ist.

Fig.1.

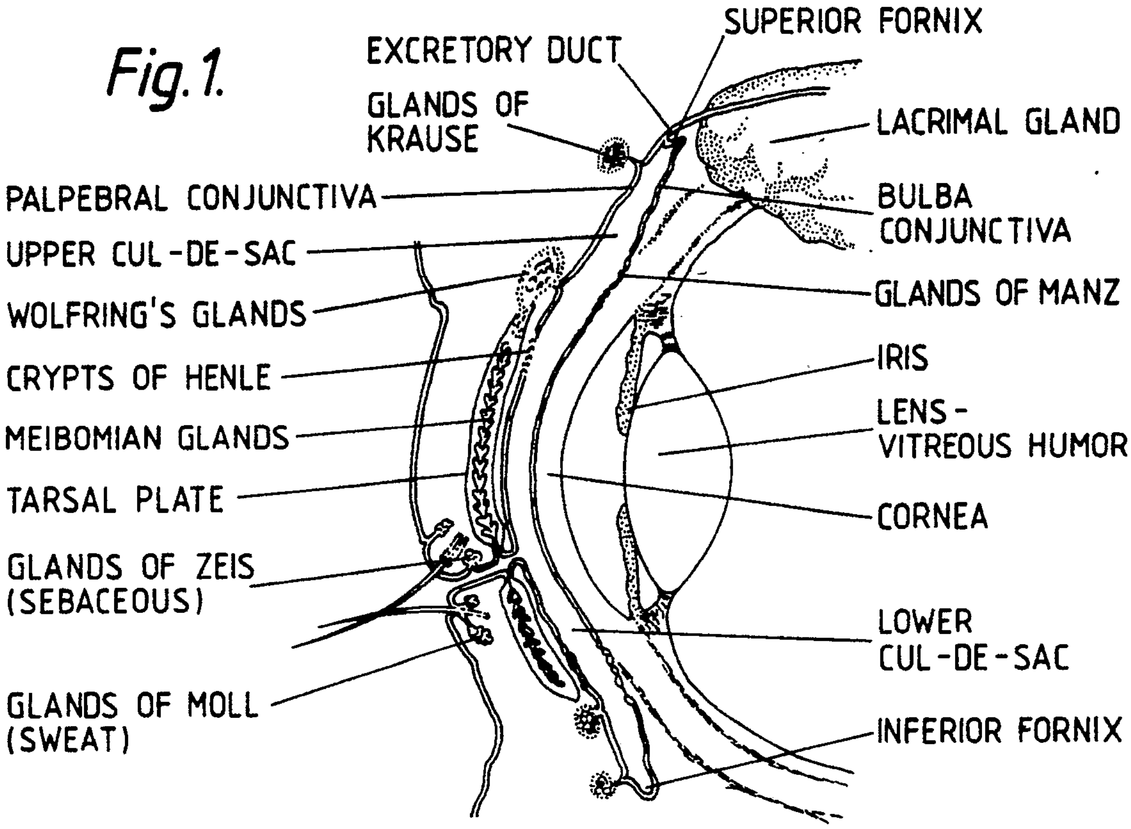
SUPERIOR FORNIX
EXCRETORY DUCT
GLANDS OF KRAUSE
LACRIMAL GLAND
PALPEBRAL CONJUNCTIVA
BULBA CONJUNCTIVA
UPPER CUL-DE-SAC
WOLFRING'S GLANDS
GLANDS OF MANZ
IRIS
CRYPTS OF HENLE
MEIBOMIAN GLANDS
LENS-VITREOUS HUMOR
TARSAL PLATE
CORNEA
GLANDS OF ZEIS (SEBACEOUS)
LOWER CUL-DE-SAC
GLANDS OF MOLL (SWEAT)
INFERIOR FORNIX

Fig.2.

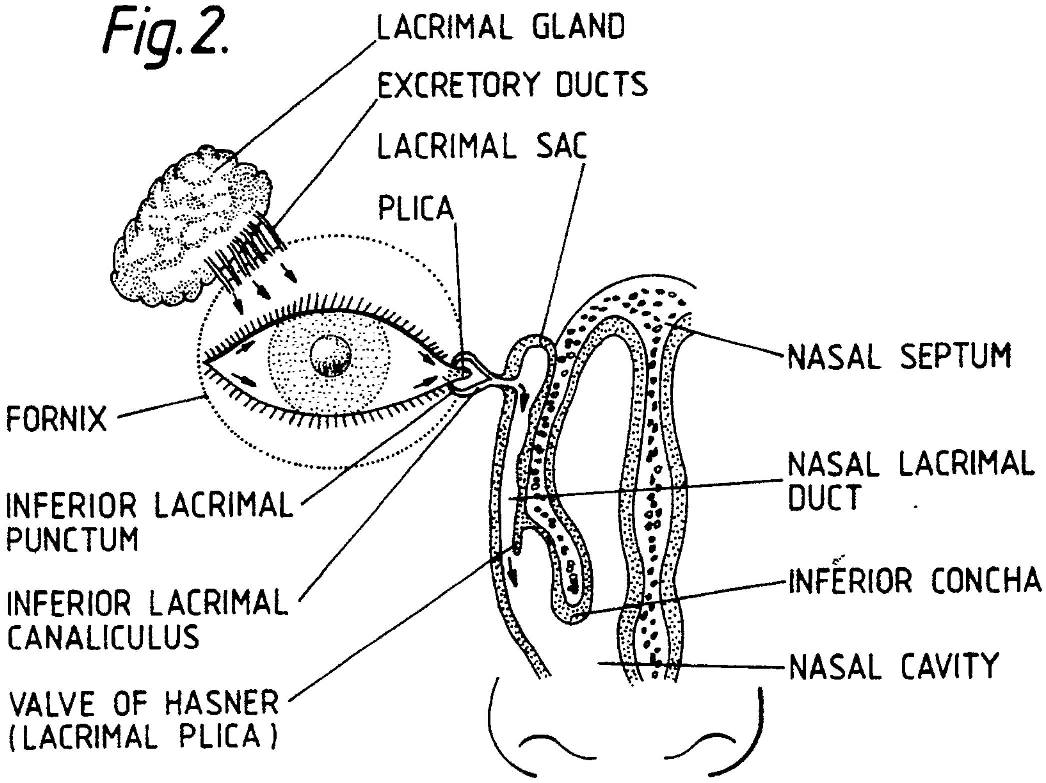
LACRIMAL GLAND
EXCRETORY DUCTS
LACRIMAL SAC
PLICA
NASAL SEPTUM
FORNIX
NASAL LACRIMAL DUCT
INFERIOR LACRIMAL PUNCTUM
INFERIOR LACRIMAL CANALICULUS
INFERIOR CONCHA
NASAL CAVITY
VALVE OF HASNER (LACRIMAL PLICA)